# DEMANDE DE BREVET EUROPEEN

(11) **EP 3 939 561 A1**
(43) Date de publication de la demande: **19.01.2022**
(21) Numéro de dépôt: 20305806.0
(22) Date de dépôt: 15.07.2020
(51) Int. Cl.: A61K 8/67, A61Q 7/00, A61P 17/04

(54) **COMPOSITION POUR FAVORISER LA POUSSE DU CHEVEU**

(71) Demandeur: Zbida, Najoi, 94600 Choisy-le-Roi (FR)
(72) Inventeur: Zbida, Najoi, 94600 Choisy-le-Roi (FR)
(74) Mandataire: Vial, Lionel

(57) **Abrégé**

La présente invention concerne une composition comprenant des vitamines A, B, C et E pour une utilisation dans une méthode pour favoriser la pousse du cheveu chez un individu.

## Description

### Domaine de l'invention

L'invention concerne une composition utile pour favoriser la pousse du cheveu.

### Arrière-plan technique

Le plasma riche en plaquettes (PRP) a récemment émergé comme un traitement prometteur pour favoriser la repousse du cheveu.
Ainsi, Gentile et al. (2015) Cells Translational Medicine 4:1317-1323 ont montré que l'administration, en autologue, de PRP, en trois séances espacées de 30 jours, à des individus atteints d'alopécie androgénique masculine à une dose de 0,1 mL/cm² injectée dans la peau du crâne à traiter, permettait en particulier d'obtenir une augmentation de la densité moyenne de cheveux (+45,9 cheveux/cm²) par rapport à la situation avant traitement.
Toutefois, l'administration de PRP ne permet pas toujours de favoriser la pousse du cheveu de manière suffisante.
En effet, Ayatollahi et al. (2017) Indian Dermatology Online Journal 8:460-464 rapportent que l'administration de PRP a des individus masculins atteints d'alopécie androgénique en 5 séances espacées de 2 semaines par injection intradermique à une dose de 0,05 mL par site d'injection, ne permettait que d'obtenir une augmentation non significative de la densité moyenne de cheveux (+18,9 cheveux/cm²).
C'est donc un objet de l'invention que d'améliorer la prise en charge de la pousse du cheveu par injection de PRP.

### Résumé de l'invention

La présente invention découle de la mise en évidence inattendue, par l'inventeur, qu'une composition à bases des vitamines A, B, C et E est utile pour favoriser la pousse des cheveux, en particulier chez des individus traités par injection de plasma riche en plaquettes (PRP).
Ainsi, la présente invention concerne une composition comprenant des vitamines A, B, C et E pour une utilisation dans une méthode pour favoriser la pousse du cheveu chez un individu.
La présente invention concerne également une méthode, notamment cosmétique, pour favoriser la pousse du cheveu, comprenant l'administration d'une composition comprenant des vitamines A, B, C et E à un individu, dans laquelle l'individu ne souffre pas d'une perte de cheveux pathologique.
La présente invention concerne également une composition à base de vitamines A, B C et E, notamment destinée à favoriser la pousse, ou la repousse, des cheveux, comprenant :
- de la vitamine A à une concentration de 5% m/m à 10% m/m ;
- l'une au moins des vitamines B1, B3, B6, B9 et B12 aux concentrations suivantes :
   - Vitamine B1 : de 0,025% m/m à 0,075% m/m ;
   - Vitamine B3 : de 0,0025% m/m à 0,0075% m/m ;
   - Vitamine B6 : de 0,025% m/m à 0,075% m/m ;
   - Vitamine B9 : de 0,0125% m/m à 0,0375% m/m ;
   - Vitamine B12 : de 0,00125% m/m à 0,00375% m/m;
- de la vitamine C à une concentration de 5% m/m à 15% m/m ;
- de la vitamine E à une concentration de 1% m/m à 2% m/m.

### Description de l'invention

A titre préliminaire, on rappellera que l'expression « consistant en » signifie « constitué de », c'est-à-dire que lorsqu'un objet « consiste en » un élément ou plusieurs éléments, l'objet ne peut pas comprendre d'autres éléments que ceux mentionnés. A contrario, le terme « comprenant » signifie « incluant », « contenant » ou « englobant », c'est-à-dire que lorsqu'un objet « comprend » un élément ou plusieurs éléments, d'autres éléments que ceux mentionnés peuvent également être compris dans l'objet. Autrement dit, lorsqu'un objet « comprend » un élément ou plusieurs éléments, il est constitué du ou des plusieurs éléments et éventuellement d'autres éléments que ceux-ci.
Comme on l'entend ici, une méthode est dite « cosmétique» si elle ne vise pas à prévenir ou traiter une maladie, en particulier si elle vise à favoriser la pousse du cheveu chez un individu ne souffrant pas, ou n'étant pas à risque, d'une chute ou d'une perte de cheveux pathologique, c'est-à-dire d'une chute ou d'une perte de cheveux due à une maladie. En particulier, une méthode cosmétique pour favoriser la pousse du cheveu est utile pour prévenir ou traiter la chute, ou la perte, non pathologique des cheveux, notamment une chute, ou une perte, de cheveux naturelle. Une chute de cheveux est généralement considérée comme pathologique si un individu perd en moyenne plus de 100 cheveux par jour pendant au moins plusieurs jours, notamment pendant une période pouvant aller jusqu'à deux mois.

### Pousse du cheveu

Comme on l'entend ici l'expression « pousse du cheveu », synonyme de « pousse des cheveux », inclut la « repousse » du cheveu ou des cheveux, chez des individus ayant subi une perte ou une chute de cheveux.
Dans un mode de réalisation préféré de l'invention, la méthode, notamment cosmétique, pour favoriser la pousse du cheveu, selon l'invention est une méthode de prévention ou de traitement de la chute ou de la perte de cheveux, pathologique ou non pathologique.
Lorsque la chute, ou la perte, de cheveux selon l'invention est pathologique, la méthode selon l'invention est une méthode de prévention ou de traitement de la maladie à l'origine de la chute, ou de la perte, de cheveux, notamment d'une alopécie, plus particulièrement sélectionnée dans le groupe constitué de l'alopécie androgénique, de l'alopécie post-ménopausique, de l'alopécie aiguë, de l'alopécie en plaque et de l'alopécie congénitale.

### Individu

L'individu selon l'invention est un mammifère, de préférence un humain. Il peut s'agir d'un homme ou d'une femme. De préférence, l'individu selon l'invention est âgé d'au moins 20, 30, 40, 50, 60, 70, 80 ou 90 ans.

Dans un mode de réalisation préféré de l'invention, l'individu est atteint ou à risque d'une perte de cheveux due à une maladie, autrement dit d'une chute de cheveux pathologique.
De préférence, l'individu est ainsi atteint ou à risque d'une perte de cheveux due à une maladie, en particulier à une alopécie, plus particulièrement sélectionnée dans le groupe constitué de l'alopécie androgénique, de l'alopécie post-ménopausique, de l'alopécie aiguë, de l'alopécie en plaque et de l'alopécie congénitale.
Comme on l'entend ici, l'expression «alopécie androgénique» est considérée synonyme d'alopécie androgénétique, notamment héréditaire.
L'alopécie aiguë englobe notamment l'alopécie consécutive à un traitement médical, notamment par chimiothérapie ou par rayonnement, l'alopécie consécutive à un stress, l'alopécie carentielle, notamment due à une carence alimentaire ou une carence en fer ou l'alopécie consécutive à des troubles hormonaux.
L'alopécie en plaque, également nommée alopecia areata ou pelade, peut notamment atteindre toute la tête, il s'agit alors d'alopecia areata totalis, voire tout le corps, il s'agit alors d'alopecia areata universalis.
De préférence, l'individu selon l'invention est atteint de calvitie, c'est-à-dire qu'il souffre d'une perte ou d'une chute de cheveux localisée sur le front, au-dessus des tempes et au sommet du crâne.
Dans un autre mode de réalisation préféré de l'invention, l'individu selon l'invention ne souffre pas d'une perte de cheveux pathologique. Dans ce cas, la méthode pour favoriser la pousse du cheveu est alors une méthode cosmétique pour favoriser la pousse du cheveu, notamment pour prévenir ou traiter la chute, ou la perte, de cheveux chez l'individu.
De préférence, l'individu selon l'invention a subi une greffe de cheveux. Dans ce cas, la méthode pour favoriser la pousse du cheveu selon l'invention vise en particulier à favoriser la pousse des cheveux greffés.
De préférence, l'individu selon l'invention est traité par administration de plasma riche en plaquettes (PRP), c'est-à-dire qu'il a été traité par administration de PRP moins de trois semaine avant qu'on lui administre la composition selon l'invention. En particulier, l'administration de PRP peut être réalisée moins de 10 jours avant qu'on lui administre la composition selon l'invention, voire le même jour.

Le plasma riche en plaquette, ou PRP, est bien connu de la personne du métier. Il est notamment décrit dans les articles Gentile et al. (2015) Cells Translational Medicine 4:1317-1323 et Ayatollahi et al. (2017) Indian Dermatology Online Journal 8:460-464. Il peut notamment être obtenu en centrifugeant du sang total, par exemple 4 mL, dans un tube sous vide traité par un anticoagulant, à une vitesse de rotation de l'ordre de 1000 g ; environ 2 mL de PRP sont ainsi obtenus. Le PRP peut être administré par injection, intradermique ou sous cutanée, dans le cuir chevelu, c'est-à-dire dans la peau du crâne. La zone de peau à traiter peut être atteinte, ou être à risque, de perte, ou de chute, de cheveux. De préférence, on injecte le PRP à une dose de l'ordre de 0,1 mL par cm² de peau à traiter. De préférence, le PRP administré est un PRP autologue, c'est-à-dire qu'il est préparé à partir du sang de l'individu auquel il est administré.

### Administration

De préférence, la composition selon l'invention est administrée par application topique sur le cuir chevelu.
De préférence, la composition selon l'invention est administrée à une dose de 1 à 10 mL. Généralement, l'ensemble de la zone à traiter du cuir chevelu est traitée par administration de la composition selon l'invention en une séance. De préférence, un individu est soumis au moins 1, 2, 3, 4, 5, 10 ou 20 séances d'administration de la composition selon l'invention, chaque séance étant séparée par au moins 1, 2, 3, 4, 5, 6, 7, 8, 9,10, 15, 30, 45 ou 60 jours.
De préférence, la composition selon l'invention est administrée à l'individu en combinaison avec du plasma riche en plaquettes (PRP).
Comme on l'entend ici, "combiné" ou "en combinaison" signifie que la composition selon l'invention, est administrée en même temps que le PRP, soit ensemble, c'est-à-dire au même site d'administration, ou séparément, ou à des moments différents, sous réserve que la période pendant laquelle la composition selon l'invention exerce son effet biologique sur l'individu et la période pendant laquelle le PRP exerce son effet biologique sur l'individu, se recoupe au moins partiellement, notamment pour exercer un effet synergique.
De préférence, après administration de la composition selon l'invention, le cuir chevelu de l'individu est traité par micro-perforation, notamment à l'aide d'un dispositif à micro-aiguilles. De tels dispositifs à micro-aiguilles, tels que le DERMAPEN^{®}, sont bien connus de la personne du métier. Avantageusement, le traitement par micro-perforation permet d'améliorer la pénétration de la composition selon l'invention dans le cuir chevelu.

### Composition

De préférence, la composition selon l'invention comprend :
- de la vitamine A à une concentration de 5% m/m à 10% m/m ;
- l'une au moins des vitamines B1, B3, B6, B9 et B12 aux concentrations suivantes :
   - Vitamine B1 : de 0,025% m/m à 0,075% m/m ;
   - Vitamine B3 : de 0,0025% m/m à 0,0075% m/m ;
   - Vitamine B6 : de 0,025% m/m à 0,075% m/m ;
   - Vitamine B9 : de 0,0125% m/m à 0,0375% m/m ;
   - Vitamine B12 : de 0,00125% m/m à 0,00375% m/m;
- de la vitamine C à une concentration de 5% m/m à 15% m/m ;
- de la vitamine E à une concentration de 1% m/m à 2% m/m.
Comme on l'entend ici, les pourcentages (%) m/m sont exprimés en grammes (g) pour 100 grammes (g) de composition.
Comme on l'entend ici, les vitamines présentes dans la composition peuvent être sous-forme d'une pro-vitamine.
De préférence, la vitamine A est sous forme de rétinol.
De préférence, la vitamine B1 est sous forme de thiamine.
De préférence, la vitamine B3 est sous forme de niacinamide.
De préférence, la vitamine B6 est sous forme de pyridoxine.
De préférence, la vitamine B9 est sous forme d'acide folique.
De préférence, la vitamine B12 est sous forme de cyanocobalamine.
De préférence, la vitamine C est sous forme d'acide ascorbique.
De préférence, la vitamine E est sous forme de tocophérol.
De préférence, la composition selon l'invention comprend l'ensemble des vitamines B1, B3, B6, B9 et B12.
De préférence, la composition selon l'invention ne comprend pas d'autre vitamine que les vitamines A, C, E et B, notamment B1, B3, B6, B9 et B12.
De préférence, la composition selon l'invention est sous forme d'une solution ou d'une suspension aqueuse.

De préférence, la composition selon l'invention est une lotion capillaire ou un shampooing.
De préférence, la composition selon l'invention comprend également au moins un autre agent sélectionné dans le groupe constitué de l'hydroxyde de sodium, notamment à une concentration de 1% m/m à 4% m/m, du phénoxyéthanol, notamment à une concentration de 0,5% m/m à 1,3% m/m, de l'éthylhexylglycérine, notamment à une concentration de 0,05% m/m à 0,15% m/m, et du benzoate de sodium, notamment à une concentration de 0,05% m/m à 0,15% m/m.
De préférence, la composition selon l'invention comprend également du pentapeptide-33, notamment à une concentration de 0,1% m/m à 0,4 % m/m.
Comme on l'entend ici, le pentapeptide-33 a la même séquence peptidique que l'exorphine de gluten B5, qui est constituée de l'enchainement des acides aminés, Tyrosine (Y), Glycine (G), Glycine (G), Tryptophane (Y) et Leucine (L), dans l'orientation de l'extrémité N-terminale à C-terminale.

### Description des figures

Figures 1 et 2

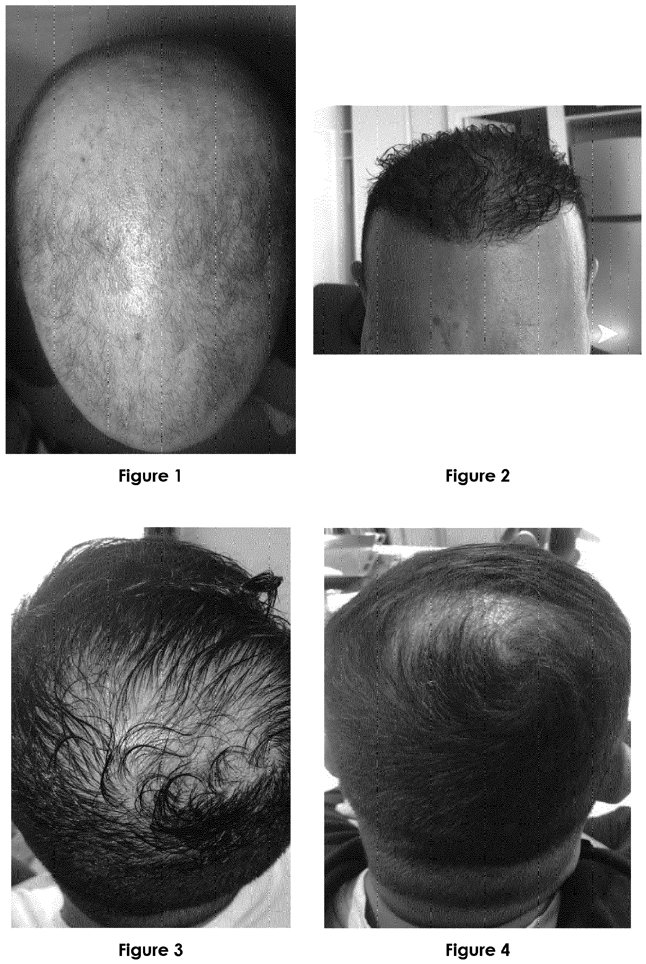

   Les figures 1 et 2 représentent des photographies du cuir chevelu d'un individu avant et après traitement avec la méthode selon l'invention.
Figures 3 et 4
   Les figures 3 et 4 représentent des photographies du cuir chevelu d'un individu avant et après traitement avec la méthode selon l'invention.
Figures 5 et 6

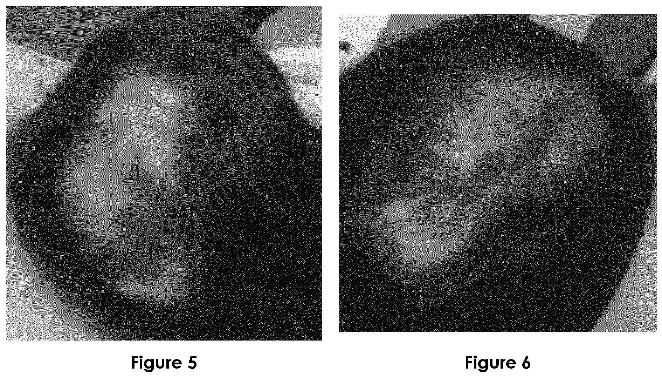

   Les figures 5 et 6 représentent des photographies du cuir chevelu d'un individu avant et après traitement avec la méthode selon l'invention.

### EXEMPLES

### Matériel

Le PRP est obtenu par centrifugation de 4 à 12 mL de sang total prélevé chez l'individu auquel il doit être administré à une vitesse de rotation de l'ordre de 1000 - 1200 g.
La composition selon l'invention est définie précédemment et comprend dans de l'eau :
- de la vitamine A à une concentration d'environ 8% m/m ;
- de la vitamine B1 à une concentration d'environ à 0,04% m/m ;
- de la vitamine B3 à une concentration d'environ 0,004% m/m ;
- de la vitamine B6 à une concentration d'environ 0,04% m/m ;
- de la vitamine B9 à une concentration d'environ 0,02% m/m ;
- de la vitamine B12 à une concentration d'environ 0,002% m/m ;
- de la vitamine C à une concentration d'environ 8% m/m ;
- de la vitamine E à une concentration d'environ 1% m/m.

### Méthode

La composition selon l'invention est administrée par application topique sur la zone de cuir chevelu de l'individu à traiter, pour un total de 1 à 10 mL de composition administrée selon la surface de la zone à traiter. Suite à l'application le cuir chevelu peut être traité à l'aide d'un dispositif à micro-aiguilles du type DERMAPEN^{®}. 2 à 3 séances d'administration espacées entre elles de 10 à 30 jours sont réalisées. Les séances incluent également l'injection intradermique de PRP sur la zone de cuir chevelu à traiter à raison d'environ 0,1 mL par cm². Par ailleurs, l'individu a pu être soumis à au moins une séance d'administration de PRP dans les 20 jours précédant la première administration de la composition selon l'invention.

### Exemple 1

La méthode de l'invention est appliquée à un individu de sexe masculin ayant subi une greffe de cheveux cinq mois auparavant.
Avant traitement, on observe que la pousse des cheveux greffés est faible (Figure 1). Deux mois plus tard, après traitement par la méthode de l'invention, la chevelure est dense, épaisse et résistante (Figure 2).

### Exemple 2

La méthode de l'invention est appliquée à un individu de sexe masculin présentant une alopécie androgénique évolutive depuis plus de 8 ans (Figure 3, avant traitement).
Après traitement, deux mois plus tard, la densité des cheveux est visiblement augmentée, l'alopécie est réduite (Figure 4).

### Exemple 3

La méthode de l'invention est appliquée à un individu de sexe féminin présentant une alopécie consécutive à une grossesse et à une carence en fer (Figure 5, avant traitement).
Après traitement, deux mois plus tard, la densité des cheveux est visiblement augmentée, l'alopécie est réduite (Figure 6).

## Revendications

1. Composition comprenant des vitamines A, B, C et E pour une utilisation dans une méthode pour favoriser la pousse du cheveu chez un individu.

2. Composition pour une utilisation selon la revendication 1, dans laquelle l'individu est traité par administration de plasma riche en plaquettes (PRP).

3. Composition pour une utilisation selon la revendication 1 ou 2, dans laquelle la composition est administrée à l'individu en combinaison avec du plasma riche en plaquettes (PRP).

4. Composition pour une utilisation selon la revendication 1, dans laquelle l'individu est atteint ou à risque d'une perte de cheveux due à une maladie.

5. Composition pour une utilisation selon la revendication 1, dans laquelle l'individu est atteint ou à risque d'une perte de cheveux due à une maladie sélectionnée dans le groupe constitué de l'alopécie androgénique, de l'alopécie post-ménopausique, de l'alopécie aiguë, de l'alopécie en plaque et de l'alopécie congénitale.

6. Composition pour une utilisation selon l'une des revendications 1 à 5, dans laquelle l'individu a subi une greffe de cheveux.

7. Composition pour une utilisation selon l'une des revendications 1 à 6, dans laquelle la composition est administrée par application topique sur le cuir chevelu.

8. Composition pour une utilisation selon l'une des revendications 1 à 7, dans laquelle après administration de la composition, le cuir chevelu de l'individu est traité par micro-perforation.

9. Composition pour une utilisation selon l'une des revendications 1 à 8, dans laquelle la composition comprend :
- de la vitamine A à une concentration de 5% m/m à 10% m/m ;
- l'une au moins des vitamines B1, B3, B6, B9 et B12 aux concentrations suivantes :
• Vitamine B1 : de 0,025% m/m à 0,075% m/m ;
• Vitamine B3 : de 0,0025% m/m à 0,0075% m/m ;
• Vitamine B6 : de 0,025% m/m à 0,075% m/m ;
• Vitamine B9 : de 0,0125% m/m à 0,0375% m/m ;
• Vitamine B12 : de 0,00125% m/m à 0,00375% m/m;
- de la vitamine C à une concentration de 5% m/m à 15% m/m ;
- de la vitamine E à une concentration de 1% m/m à 2% m/m.

10. Méthode cosmétique pour favoriser la pousse du cheveu, comprenant l'administration d'une composition comprenant des vitamines A, B, C et E à un individu, dans laquelle l'individu ne souffre pas d'une perte de cheveux pathologique.

11. Méthode cosmétique selon la revendication 11, pour prévenir ou traiter la chute de cheveux chez l'individu.

12. Méthode cosmétique selon la revendication 10 ou 11, dans laquelle l'individu l'individu est traité par administration de plasma riche en plaquettes (PRP).

13. Méthode cosmétique selon l'une des revendications 10 à 12, dans laquelle dans laquelle la composition comprend :
- de la vitamine A à une concentration de 5% m/m à 10% m/m ;
- l'une au moins des vitamines B1, B3, B6, B9 et B12 aux concentrations suivantes :
• Vitamine B1 : de 0,025% m/m à 0,075% m/m ;
• Vitamine B3 : de 0,0025% m/m à 0,0075% m/m ;
• Vitamine B6 : de 0,025% m/m à 0,075% m/m ;
• Vitamine B9 : de 0,0125% m/m à 0,0375% m/m ;
• Vitamine B12 : de 0,00125% m/m à 0,00375% m/m;
- de la vitamine C à une concentration de 5% m/m à 15% m/m ;
- de la vitamine E à une concentration de 1% m/m à 2% m/m.

14. Composition à base de vitamines A, B C et E, comprenant :
- de la vitamine A à une concentration de 5% m/m à 10% m/m ;
- l'une au moins des vitamines B1, B3, B6, B9 et B12 aux concentrations suivantes :
• Vitamine B1 : de 0,025% m/m à 0,075% m/m ;
• Vitamine B3 : de 0,0025% m/m à 0,0075% m/m ;
• Vitamine B6 : de 0,025% m/m à 0,075% m/m ;
• Vitamine B9 : de 0,0125% m/m à 0,0375% m/m ;
• Vitamine B12 : de 0,00125% m/m à 0,00375% m/m;
- de la vitamine C à une concentration de 5% m/m à 15% m/m ;
- de la vitamine E à une concentration de 1% m/m à 2% m/m.

15. Kit comprenant :
- une composition à base de vitamines A, B C et E selon la revendication 14, et
- un dispositif à micro-aiguilles.
